# EUROPEAN PATENT APPLICATION

(11) **EP 3 777 722 A1**
(43) Date of publication of application: **17.02.2021**
(21) Application number: 19778269.1
(22) Date of filing: 13.03.2019
(51) Int. Cl.: A61B 17/22, A61F 2/95

(54) **RETRIEVAL DEVICE**

(30) Priority: 30.03.2018 JP 2018068343
(71) Applicant: KURUME UNIVERSITY, Kurume-shi Fukuoka 830-0011 (JP); TOKUSEN KOGYO CO., LTD., Ono-city Hyogo 675-1361 (JP); Medikit Co., Ltd., Tokyo, 113-0034 (JP)
(72) Inventor: FUKUMOTO Yoshihiro, Kurume-shi, Fukuoka 830-0011 (JP); NAM Kwangwoo, OnoCity, Hyogo 6751361 (JP); TANAKA Yasuomi, Tokyo 113-0034 (JP); UMEDA Masahiro, Tokyo 113-0034 (JP)
(74) Representative: Grünecker Patent- und Rechtsanwälte PartG mbB
(86) International application number: PCT/JP2019/010369
(87) International publication number: WO 2019/188308

(57) **Abstract**

Provided is a device for collection that can easily collect an object such as a stent from a lumen defined by a lumen wall. The device for collection (1) is used to collect an object such as a stent from a lumen defined by a lumen wall. The device for collection (1) includes an operation wire (2) that is inserted movably forward and backward inside a catheter (4) that is a hollow flexible tube and a snare wire (3) that is provided at the tip of the operation wire (2). The snare wire (3) is formed of one wire, which includes a first loop (3a) at the base end side, a second loop (3b) at the leading end side, and an intersection (3c) of the first loop (3a) with the second loop (3b). The snare wire (3) is double looped, in which the first loop (3a) and the second loop (3b) are approximately concentrically located adjacent to each other. The intersection (3c) is located at leading end side of the first loop (3a). The operation wire (2), the first loop (3a), and the second loop (3b) are located on an approximately same plain face.

## Description

### TECHNICAL FIELD

The present invention relates to a device for collection. More specifically, the present invention relates to a device for collection used to collect an object such as a stent from a lumen defined by a lumen wall.

### BACKGROUND

Conventionally, the technology to collect a migrated stent with a snare (device for collection) has been known (for example, refer to Patent Document 1 and Non-Patent Document 1).

Patent Document 1 describes that the stent is pulled in a medical tube by hooking one end of the stent on a looped snare when a stent needs to retry to be inserted because the stent is inappropriately placed (refer to the paragraphs [0051], [0052], etc.).

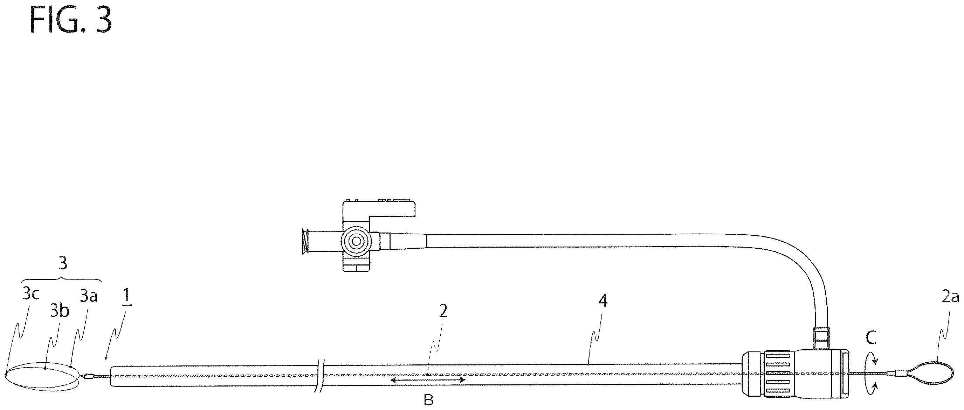

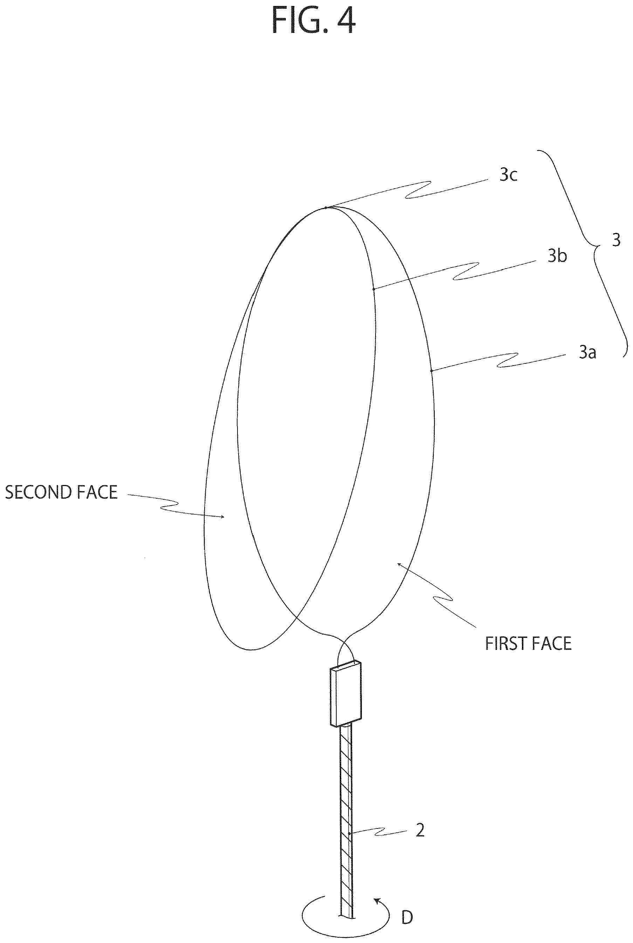

Non-Patent Document 1 describes that the pancreatic duct stent migrated in the pancreatic duct in the center of the pancreatic body is collected with a looped snare (refer to Figures 3 and 4, etc.).

### Citation List

### Patent Literature

Patent Document 1: JP 2017-070512 A

### Non-patent Literature

Non-Patent Document 1: Asunaga KATO, et al., "A Case of Migrated Pancreatic Stent Removed by a Vascular Interventional Device," online, November, 2013, Japan Gastroenterological Endoscopy Society, retrieved on February 20, 2018, from the URL https://www.jstage.jst.go.jp/article/gee/55/11/55_3598/_pdf

### SUMMARY

However, a conventional snare (device for collection) requires a skilled technique to hook a stent and takes a long time to collect a stent.

An objective of the present invention is to provide a device for collection that can easily collect an object such as a stent from a lumen defined by a lumen wall without a skilled technique.

According to the first aspect of the present invention, the device for collection that is used to collect an object from a lumen defined by a lumen wall, includes:
an operation wire that moves forward and backward inside a hollow flexible tube; and
a snare wire that is provided at the tip of the operation wire, in which
the snare wire is formed of one wire and includes:
   a first loop at the base end side,
   a second loop at the leading end side, and
   an intersection of the first loop with the second loop,
the first loop and the second loop are approximately concentrically located adjacent to each other to form a double loop,
the intersection is located at the leading end side of the first loop, and
the operation wire, the first loop, and the second loop are located on an approximately same plain face.

According to the first aspect of the present invention, the device for collection has the following function effect. The operation wire is inserted and loaded in a hollow flexible tube, the flexible tube is inserted in a lumen from, for example, the dissected base of a patient's leg, and the tip is brought to near an object such as stent. Next, the bottom end of the flexible tube is held with the left hand, and then the base end of the operation wire is held with the right hand, and then the snare wire is pushed out of the tip of the flexible tube by moving the operation wire forward. Next, the operation wire is moved forward and backward to hook the snare wire on an object such as a stent. The object such as a stent only has to be hooked by at least any one of the first loop and the second loop. Particularly, according to the first aspect of the present invention, the first loop and the second loop are approximately concentrically located adjacent to each other.

This enables the two loops with an approximately same size to capture an object such as a stent, resulting in the increased probability of capturing an object such as a stent. Therefore, according to the first aspect of the present invention, an object such as a stent can be easily collected from a lumen defined by a lumen wall without a skilled technique.

The device for collection according to the first aspect of the present invention preferably has the below-mentioned configuration according to any one of the second to seventh aspects of the present invention.

According to the second aspect of the present invention, the operation wire is rotated in the lumen to allow the first loop and then the second loop to rotate to form a three-dimensional shape, and the first face having the first loop intersects with the second face having the second loop in the three-dimensional shape. According to the preferable configuration of the second aspect of the present invention, the intersecting two faces (loops) can capture an object such as a stent, resulting in the increased probability of capturing an object such as a stent.

According to the third aspect of the present invention, the snare wire is formed of a wire made of a shape-memory alloy. According to the preferable configuration of the third aspect of the present invention, the two loops can be put in the body in an ideal condition to capture an object such as a stent.

According to the fourth aspect of the present invention, the snare wire is formed from any one of a nickel-titanium alloy, a stainless steel, titanium, and a titanium alloy. According to the preferable configuration of the fourth aspect of the present invention, the snare wire can be achieved by meeting the required biocompatibility and hardly making itself deformed, for example, crashed or folded even if it passes through the inside of a thin flexible tube.

According to the fifth aspect of the present invention, the diameter of the wire is from 0.1 to 0.7 mm.

According to the sixth aspect of the present invention, the each diameter of the first loop and the second loop that are formed in circles is from 10 to 70 mm. According to the preferable configuration of the sixth aspect of the present invention, the two loops can have an ideal size to capture an object such as a stent.

According to the seventh aspect of the present invention, the operation wire is formed of at least a stranded wire that is produced by twisting multiple metallic wires. According to the preferable configuration of the seventh aspect of the present invention, the operation wire can have moderate flexibility and rigidity, small axial stretchability, and torque transmissibility that can surely operates the tip by the hand operation. Therefore, the excellent device for collection that can easily bring the snare wire to, for example, a stenting position and have the excellent rotating operability of two loops can be provided.

According to the present invention, an object such as a stent can be easily collected from a lumen defined by a lumen wall without a skilled technique.

### BRIEF DESCRIPTION OF DRAWINGS

Fig. 1 is a side perspective view illustrating the configuration of the device for collection of one embodiment of the present invention.
Fig. 2 enlarges the part A enclosed by the chain double-dashed line shown in Fig. 1.
Fig. 3 is a side perspective view illustrating the state in which the operation wire that is a structural member of the device for collection of one embodiment of the present invention has been inserted movably forward and backward inside a catheter that is a hollow flexible tube.
Fig. 4 is an enlarged perspective view illustrating the snare wire with another configuration that is a structural member of the device for collection of one embodiment of the present invention.
Fig. 5 illustrates to explain aortic dissection and the method of treating the same.
Fig. 6 is a side view illustrating one example stent used to treat aortic dissection.
Fig. 7 is a side perspective view illustrating the state in which the tip of a catheter that is a hollow flexible tube has reached near a treated area after the device for collection of one embodiment of the present invention was placed in the catheter.
Fig. 8 is a side perspective view illustrating the state in which the snare wire that is a structural member of the device for collection of one embodiment of the present invention has been pushed out of the tip of the catheter in the state of Fig. 7.
Fig. 9 is a side perspective view illustrating the state in which the snare wire that is a structural member of the device for collection of one embodiment of the present invention has been hooked on a stent.
Fig. 10 enlarges the part G enclosed by the chain double-dashed line shown in Fig. 9.
Fig. 11 is a side perspective view illustrating the state in which a stent has been pulled in a catheter with the device for collection of one embodiment of the present invention.
Fig. 12 is an enlarged perspective view illustrating the snare wire with another configuration that is a structural member of the device for collection of one embodiment of the present invention.

### DETAILED DESCRIPTION

The present invention will be more specifically described below with reference to the preferable embodiments. However, these are illustrative only, and the present invention is not limited thereto.

### Configuration of device for collection

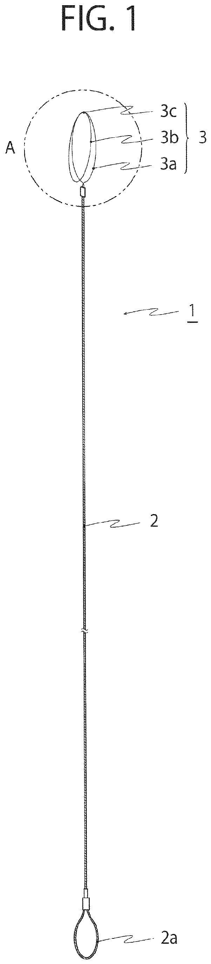

The configuration of the device for collection of one embodiment of the present invention is described below with reference to Figs. 1 to 4.

More specifically, the device for collection 1 shown in Figs. 1 to 3 is used to collect an object such as a stent from a lumen defined by a lumen wall. This device for collection 1 includes an operation wire 2 and a snare wire 3. The objects to be collected are, for example, a damaged part of catheter, an artificial blood vessel, a prosthetic valve as well as a stent. The operation wire 2 is inserted movably forward and backward inside a catheter 4 that is a hollow flexible tube (refer to the double-headed arrow B of Fig. 3). An operation ring 2a is formed at the bottom of the operation wire 2. The operation ring 2a is held to move the operation wire 2 forward and backward (refer to the double-headed arrows B and C of Fig. 3) and rotate it inside the catheter 4. The snare wire 3 is provided at the tip of the operation wire 2. The snare wire 3 is formed of one wire that is circular in cross-section. The snare wire 3 is double looped in the absence of external forces, which includes a first loop 3a at the base end side, a second loop 3b at the leading end side, and an intersection 3c of the first loop 3a with the second loop 3b. In this case, the intersection 3c is located at leading end side of the first loop 3a. At the intersection 3c, parts of the wire intersect with each other in a state of nature while no external force is being applied. The intersection 3c, the first loop 3a, and the second loop 3b may not exist if the wire is twisted.

To collect an object such as a stent, the object such as a stent only has to be hooked by at least any one of the first loop 3a and the second loop 3b. Therefore, according to the configuration of the device for collection 1 of this embodiment, an object such as a stent can be easily collected from a lumen defined by a lumen wall without a skilled technique.

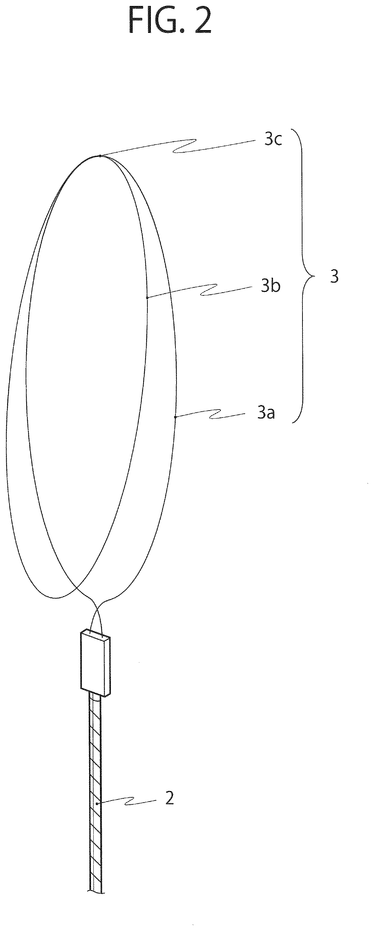

The configuration of the device for collection of this embodiment is more specifically described below. As shown in Figs. 1 to 3, the first loop 3a and the second loop 3b are approximately concentrically located adjacent to each other in a state of nature while no external force is being applied. According to the configuration, the two loops 3a and 3b an approximately same size can capture an object such as a stent, resulting in the increased probability of capturing an object such as a stent. Moreover, when the operation wire 2 is rotated in the direction of the arrow D of Fig. 4 in a lumen from the state of Fig. 2 to that of Fig. 4, the snare wire 3 rotates from the first loop 3a of the base end side (hand side). When the operation wire 2 is further rotated, the second loop 3b rotates. Then, a three-dimensional shape in which the first face having the first loop 3a intersects with the second face having the second loop 3b is formed. As the result, the intersecting two faces (loops) can capture an object such as a stent, resulting in the increased probability of capturing an object such as a stent. When three-dimensional shape is formed, the angle between the first loop 3a (first face) and the second loop 3b (second face) is preferably 80° or more and 100° or less. According to the preferable configuration, when the operation wire 2 is rotated to capture an object such as a stent, the two loop faces appropriately intersect with each other. The angle between the first loop 3a and the second loop 3b may be approximately 0° or more and 45° or less, preferably 0° or more and 20° or less. When the operation wire 2 is rotated, the degree between of the two loops 3a and 3b is increased. Alternatively, the first loop 3a and the second loop 3b are distorted to form the snare wire 3 with a different three-dimensional shape from the above-mentioned one by pulling the snare wire 3 in the catheter 4 from the state of Fig. 3 and pushing it out of the tip of the catheter 4 again.

The snare wire 3 is formed of a wire made of a shape-memory alloy. According to the configuration, the two loops 3a and 3b can be put in the body in an ideal condition to capture an object such as a stent. When the snare wire 3 is pushed out of the tip of the catheter 4 in a lumen and warmed to a body temperature, the first loop 3a and the second loop 3b come close to each other and then are arranged approximately concentrically (refer to Figs. 1 to 3).

When the snare wire 3 is pushed out of the tip of the catheter 4 in a lumen and warmed to a body temperature, the first face having the first loop 3a and the second face having the second loop 3b intersect with each other to form a three-dimensional shape. In other words, the three-dimensional shape may be naturally formed, not by rotating the two loops in the body. In this case, an object such as a stent is easily collected before the two loops 3a and 3b rotate. Therefore, an object such as a stent can be easily collected without rotation, only with forward and backward movements.

The material of the wire is preferably any one of a nickel-titanium alloy, a stainless steel, titanium, and a titanium alloy. According to the preferable configuration, the snare wire 3 can be achieved by meeting the required biocompatibility and hardly making itself deformed, for example, crashed or folded even if it passes in a thin catheter 4. The stainless steel is preferable SUS304 or SUS316L. SUS304 is available and relatively inexpensive. SUS316L has the best corrosion resistance among in the stainless steels. The diameter of the wire is from 0.1 to 0.7 mm, particularly preferably from 0.2 to 0.5 mm.

The each diameter of the first loop 3a and the second loop 3b formed in circles is from 10 to 70 mm, particularly preferably from 20 to 50 mm. According to the preferable configuration, the two loops 3a and 3b can have an ideal size to capture an object such as a stent.

The operation wire 2 is formed of a stranded wire that is produced by twisting multiple metallic wires such as stainless steel wires. According to the configuration, the operation wire 2 can have moderate flexibility and rigidity, small axial stretchability, and torque transmissibility that can surely operate the tip by the hand operation. Therefore, the excellent device for collection that can easily bring the snare wire 3 to, for example, a stenting position and have the excellent rotating operability of the two loops 3a and 3b can be provided.

### Method of using device for collection

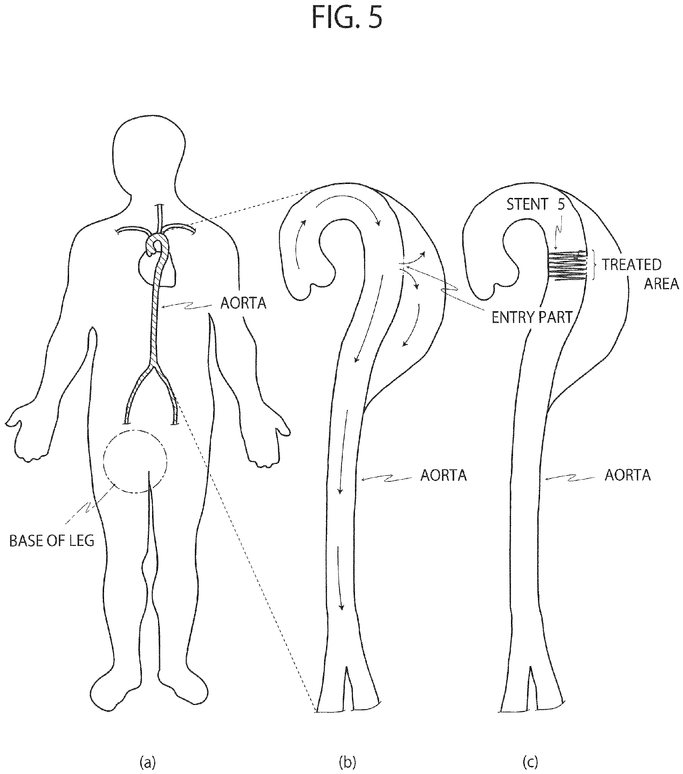

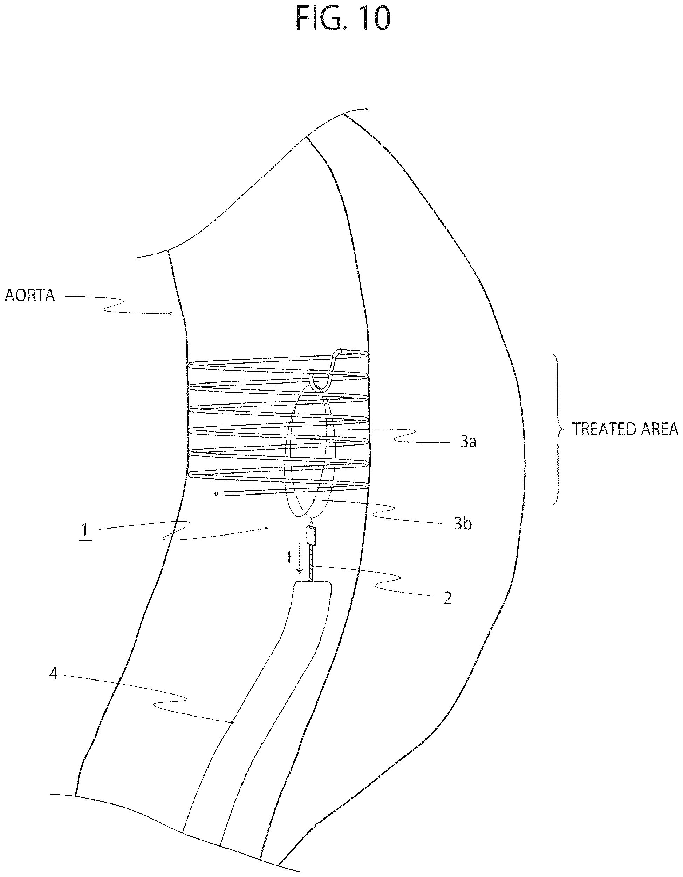

The method of using the device for collection of one embodiment of the present invention is explained below with reference to Figs. 5 to 11, giving an example where the stent used to treat aortic dissection is placed and collected.

Aorta has a three-layered structure including adventitia, media, and intima, which has enough strength and elasticity. However, an intimal tear (entry) occurs in the intima inside for some reasons, so that blood may flow into the media outside the intima to tear an aorta in a long axis direction (refer to the diagrams (a) and (b) of Fig. 5). This is called "aortic dissection." For a treatment of aortic dissection, temporally placing a stent inside a blood vessel is proposed (refer to the diagram (c) of Fig. 5).

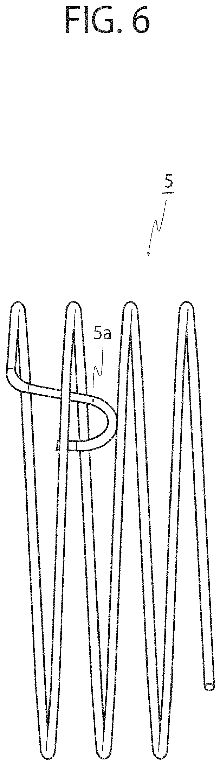

As shown in Figs. 5 and 6, the stent 5 is delivered from the dissected base of a patient's leg through the inside of a catheter 4 and is a coil member that supports the inner wall of an aorta from the inside while being placed at a treated area in an aorta. The stent 5 takes an elongate form to elongate along the inside of the catheter 4 by pulling the stent 5 and a coil form to support the inner wall of an aorta from the inside by delivering the stent 5 into a blood vessel from the tip of the catheter 4. The stent 5 also has a hook 5a for collection at its base end.

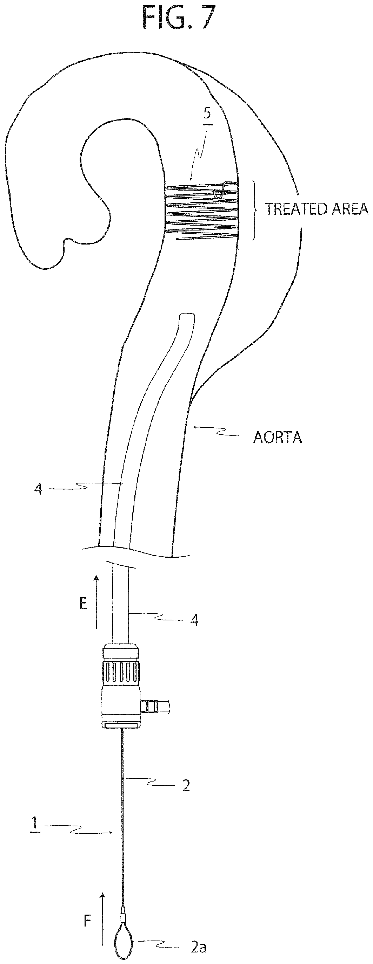

After the treated area has been cured or when a temporal stent is replaced with a permanent one, the stent 5 that completed its role is collected as described below. The stent 5 is collected while being monitored by angiography. As shown in the diagram (a) of Fig. 5 and Fig. 7, after the device for collection 1 is loaded inside of a catheter 4, the catheter 4 is first inserted in a blood vessel from the dissected base of a patient's leg (refer to the arrow E of Fig. 7), and the tip is brought to near the treated area in an aorta.

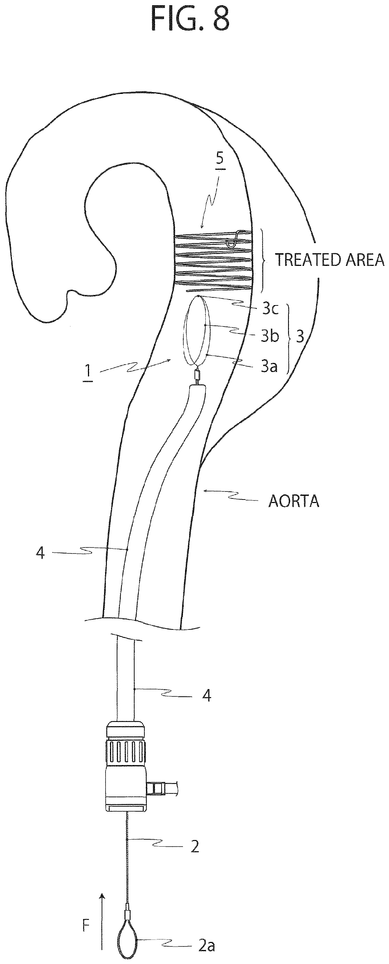

As shown in Figs. 7 and 8, the bottom of the catheter 4 is held with the left hand, and the operation ring 2a at the bottom of the operation wire 2 moves the operation wire 2 forward (refer to the arrow F of Figs. 7 and 8) with the right hand to push the snare wire 3 out of the tip of the catheter 4. At this point, the snare wire 3 is warmed to a body temperature, and the first loop 3a and the second loop 3b come close to each other and then are arranged approximately concentrically.

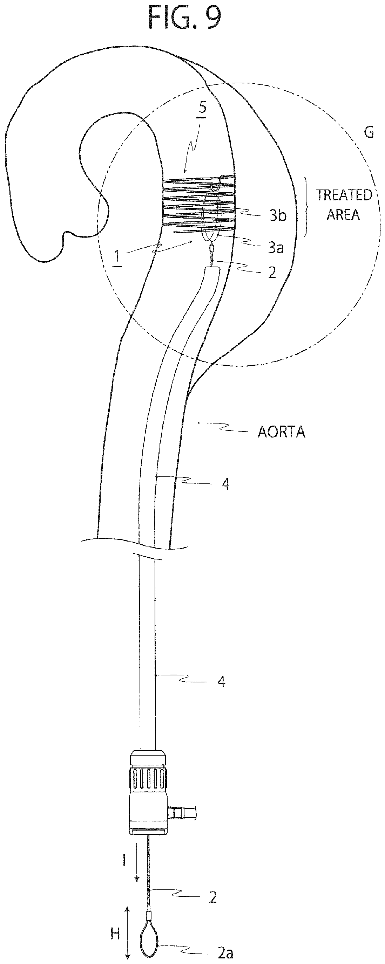

Next, as shown in Figs 8 to 10, the operation wire 2 is moved forward (refer to the double-headed arrow H of Fig. 9) to hook the snare wire 3 with the hook 5a of the stent 5 (refer to Fig. 6). The snare wire 3 is formed of one wire and double looped, which includes a first loop 3a at the base end side, a second loop 3b at the leading end side and an intersection 3c of the first loop 3a with the second loop 3b. The hook 5a of the stent 5 only has to be hooked by at least any one of the first loop 3a and the second loop 3b. Therefore, the snare wire 3 can be easily hooked by the hook 5a of the stent 5 without a skilled technique. Particularly, the first loop 3a and the second loop 3b that come close to each other and then are arranged approximately concentrically enables the two loops 3a and 3b with an approximately same size to capture a stent 5, resulting in the increased probability of capturing a stent 5.

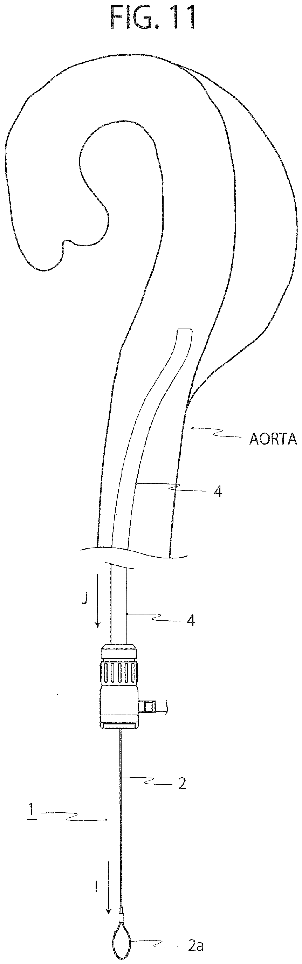

As shown in Figs. 9 to 11, the bottom of the catheter 4 is held with the left hand, and the operation ring 2a at the bottom of the operation wire 2 moves the operation wire 2 backward (refer to the arrow I of Figs. 9 to 11) with the right hand to pull the stent 5 in the catheter 4. As the result, the stent 5 moves backward inside the catheter 4, transforming itself from the coil form to the elongate form and is collected outside the body. Finally, the catheter 4 is removed from the dissected base of a patient's leg (refer to the arrow J of Fig. 11). The collection of the stent 5 that was used to treat aortic dissection is completed here.

This embodiment has been explained, giving an example of the catheter 4 used that is a hollow flexible tube. However, the present invention is not necessarily limited to such an example. For example, a sheath can be used as the hollow flexible tube.

Moreover, this embodiment has been explained, giving an example of the snare wire 3 formed of a wire with a circular cross-section. However, the present invention is not necessarily limited to such an example. The wire may have a modified cross-section, in the same way as a flat wire.

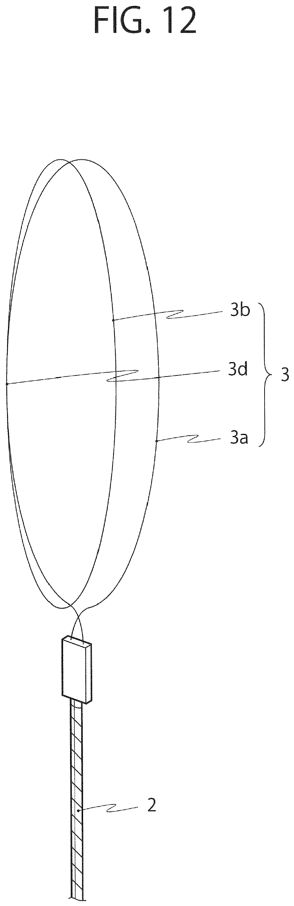

Moreover, this embodiment has been explained, giving an example where the intersection 3c of the first loop 3a with the second loop 3b is located at the leading end side of the first loop 3a. However, the present invention is not necessarily limited to such an example. For example, as shown in Fig. 12, the intersection 3c of the first loop 3a with the second loop 3b may be located at the right or left side of the first loop 3 a.

Moreover, this embodiment has been explained, giving an example where the snare wire 3 is double looped in the absence of external forces, which includes a first loop 3a at the base end side, a second loop 3b at the leading end side, and an intersection 3c of the first loop 3a with the second loop 3b, and the first loop 3a and the second loop 3b come close to each other and then are arranged approximately concentrically. In other words, this embodiment has been explained, giving an example where the first loop 3a and the second loop 3b has an approximately same size (diameter) in a state of nature while no external force is being applied. However, the present invention is not necessarily limited to such an example. The first loop 3a may be smaller or larger than the second loop 3b and may be not concentrically located in a state of nature while no external force is being applied.

Moreover, this embodiment has been explained, giving an example of only the operation wire 2 formed of a stranded wire that is produced by twisting multiple metallic wires. However, the present invention is not necessarily limited to such an example. The operation wire and the snare wire may be formed of a stranded wire that is produced by twisting multiple metallic wires.

Moreover, this embodiment has been explained, giving an example of the coiled stent used to treat aortic dissection. However, the device for collection of the present invention is not necessarily limited to such an example. The device for collection of the present invention can be used to, for example, collect a tubular mesh stent as described in Patent Document 1. Moreover, the device for collection of the present invention can be used to, for example, remove foreign body such as a temporal inferior vena cava filer or a torn catheter from a blood vessel. Even in this case, foreign body can be efficiently collected and removed from a blood vessel by getting the snare wire 3 to take the form as shown in Figs. 1 to 3 or a three-dimensional form.

### DESCRIPTION OF REFERENCE NUMBERS

1 Device for collection
2 Operation wire
2a Operation ring
3 Snare wire
3a First loop
3b Second loop
3c Intersection
4 Catheter

## Claims

1. The device for collection that is used to collect an object from a lumen defined by a lumen wall, comprising:
an operation wire that moves forward and backward inside a hollow flexible tube; and
a snare wire that is provided at the tip of the operation wire, wherein
the snare wire is formed of one wire and comprises:
a first loop at the base end side;
a second loop at the leading end side; and
an intersection of the first loop with the second loop,
the first loop and the second loop are approximately concentrically located adjacent to each other to form a double loop,
the intersection is located at the leading end side of the first loop, and
the operation wire, the first loop, and the second loop are located on an approximately same plain face.

2. The device for collection according to claim 1, wherein the operation wire is rotated in the lumen to allow the first loop and then the second loop to rotate to form a three-dimensional shape, and the first face having the first loop intersects with the second face having the second loop in the three-dimensional shape.

3. The device for collection according to claim 1 or 2, wherein the snare wire is formed of a wire made of a shape-memory alloy.

4. The device for collection according to claim 1 or 2, wherein the snare wire is formed from any one of a nickel-titanium alloy, a stainless steel, titanium, and a titanium alloy.

5. The device for collection according to any one of claims 1 to 4, wherein the diameter of the wire is from 0.1 to 0.7 mm.

6. The device for collection according to any one of claims 1 to 5, wherein the each diameter of the first loop and the second loop that are formed in circles is from 10 to 70 mm.

7. The device for collection according to any one of claims 1 to 6, wherein the operation wire is formed of at least a stranded wire that is produced by twisting multiple metallic wires.
